# EUROPÄISCHE PATENTANMELDUNG

(11) **EP 1 443 066 A2**
(43) Veröffentlichungstag der Anmeldung: **04.08.2004**
(21) Anmeldenummer: 04000957.3
(22) Anmeldetag: 19.01.2004
(51) Int. Cl.: C08G 18/44, C08G 65/34, C07C 41/34

(54) **Verfahren zur Herstellung von oligomeren aliphatischen Diolen, darauf basierenden Polycarbonatdiolen und deren Prepolymeren**

(30) Priorität: 31.01.2003 DE 10303881
(71) Anmelder: Bayer Aktiengesellschaft, 51368 Leverkusen (DE)
(72) Erfinder: Nefzger, Harmut, Dr., 50259 Pulheim (DE); Bauer, Erika, 41363 Jüchen (DE); Schmidt, Manfred, Dr., 41540 Dormagen (DE); Ruprecht, Hans-Dieter, Dr., 51061 Köln (DE); Barnes, James-Michael, 53547 Breitscheid (DE)

(57) **Zusammenfassung**

Die vorliegende Erfindung betrifft ein Herstellverfahren für oligomere aliphatische Diole, darauf basierende Polycarbonatdiole und aus ihnen erhältliche NCOterminierte Prepolymere, sowie deren Verwendung zur Herstellung von Polyurethanen.

## Beschreibung

Die vorliegende Erfindung betrifft ein Herstellverfahren für oligomere aliphatische Diole, darauf basierende Polycarbonatdiole und aus ihnen erhältliche NCO-terminierte Prepolymere, sowie deren Verwendung zur Herstellung von Polyurethanen.

Aliphatische Polycarbonat-Diole sind seit langem bekannt. Sie können aus nicht-vicinalen Diolen durch Umsetzung mit Diarylcarbonaten (DE-OS 19 15 908), Dialkylcarbonaten (DE-OS 25 55 805), Dioxolanonen (DE-OS 25 23 352), Phosgen (DE-OS 15 95 446), Bischloroformiaten (DE-OS 8 57 948) oder Harnstoff (Angew. Chem. 92 (1980) 742 hergestellt werden. Von den zahlreichen in der Literatur beschriebenen zu verwendenden Diolen haben sich in der industriellen Praxis lediglich das 1,6-Hexandiol bzw. von 1,6-Hexandiol abgeleitete Verbindungen breit durchgesetzt. So werden z.B. hochwertige Polyurethanelastomere oder auch Lacke mit Polycarbonatdiolen hergestellt, die auf 1,6-Hexandiol basieren.

Herausragend ist insbesondere die Hydrolysestabilität aus derartigen Polycarbonatpolyolen hergestellter Polyurethane. Sie übertrifft analoge Produkte aus Polyadipatpolyolen bei weitem. Reine Hexandiolpolycarbonate mit zahlenmittleren Molekulargewichten von 500 bis 5000 sind wachsartige Substanzen mit einem Erweichungstemperaturbereich von ca. 45 bis 55°C, abhängig vom Molekulargewicht. Dem entsprechend weisen die daraus hergestellten Polyurethane bei niedrigen Temperaturen einen erhöhten Schubmodul auf, d.h. sie verlieren ihre Flexibilität. Aus diesem Grunde wurden Diole entwickelt, die diesen Nachteil ausgleichen sollten. Genannt seien z.B. auf Adipinsäure basierende Oligoester (DE-AS 19 64 998), Oligoester auf Basis von Caprolacton (DE-AS 17 70 245) oder oligomere Tetraethylenglykole (DE-AS 22 21 751) und Tetrabutylenglykole. Nachteilig an diesen Bausteinen ist deren leichter hydrolysierbare Estergruppe bzw. die erhöhte Hydrophilie, die zumindest zu einem stärkeren Anquellen der daraus hergestellten PUR-Formkörper führt.

Ein weiterer Nachteil der auf Hexandiol basierenden Polycarbonatdiole ist deren vergleichsweise hohe Viskosität (ca. 5000 mPas bei 60°C und einem zahlenmittleren Molekulargewicht von 2000 g/mol). Sie kann bei der Verarbeitung zu Polyurethan-Formkörpern zu Problemen führen.

Entsprechend der Lehre von US-A 4,808,691 können die genannten Nachteile dadurch überwunden werden, dass man oligomere Hexandiole mit Diphenylcarbonat in einem solchen stöchiometrischen Verhältnis umsetzt, dass Polycarbonatdiole mit zahlenmittleren Molekulargewichten von 500 bis 12000, bevorzugt 700 bis 6000, resultieren, wobei der Oligomerisierungsgrad des Ethers so gewählt wird, dass das Verhältnis Ether- zu Carbonatgruppen von 5:1 bis 1:5, bevorzugt 3:1 bis 1:3 beträgt.

In der industriellen Praxis erweist sich jedoch die in US-A 4,808,691 offenbarte Herstellmethode für oligomere Hexandiole als zeitaufwendig, arbeitsintensiv und daher kostenträchtig. Die dort detailliert beschriebene Methode erfordert die destillative Abtrennung von Reaktionswasser, wobei auch Schleppmittel und Katalysatoren verwendet werden. Als bevorzugter Katalysator wird Naphthalin-1,5-disulfonsäure genannt. Als Schleppmittel kommen Toluol, Xylol, Gasölfraktionen, Cyclohexan, Chlorbenzol und das auch als Nebenprodukt sich bildende Oxepan zur Anwendung, wodurch dessen Bildung etwas unterdrückt werden kann.

Die Aufarbeitung nach Erreichen des gewünschten Oligomerisierungsgrades, erkennbar an der gebildeten Wassermenge, erfolgt gemäß US-A 4,808,691 derart, dass die Reaktionsmischung auf 100°C abgekühlt wird und in der Reaktionsmischung enthaltene Sulfonsäureester durch Zusatz von 5 - 10% Wasser in 1-3 Stunden hydrolysiert werden. Der freigesetzte Katalysator wird mit wässrigem Alkali oder Ammoniak neutralisiert, Wasser und andere flüchtige Bestandteile werden abdestilliert und der als Salz abgeschiedene Katalysator abfiltriert.

In der industriellen Praxis zeigen sich bei derartiger Vorgehensweise jedoch zwei gravierende Nachteile: Das Salz fällt in einer Form an, die nur unter größtem Zeitaufwand abgetrennt werden kann. Typische Filtrationszeiten für einen Ansatz von 3 Tonnen liegen in einer Größenordnung von 30 Std., wobei jede Stunde ein Filterwechsel erforderlich ist. Kleinere Ansätze von z.B. 200 kg erfordern Filtrationszeiten von ca. 5 Std.

Die Verwendung gröberer Filtermaterialien, die zwar eine schnellere Filtration ermöglichen aber zu einer weniger vollständigen Abtrennung des Salzes führen, kommt nicht in Betracht, da selbst kleinste Menge dieses Salzes eine Umsetzung der erhaltenen Polyole zu Polycarbonatdiolen negativ beeinflussen.

Weiterhin zeigt sich, dass gemäß US-A 4,808,691 hergestellte oligomere Hexandiole nicht universell weiter einsetzbar sind. Sie lassen sich zwar mit Diphenylcarbonat unter Katalyse mit Bis(tributylzinn)oxid oder Dibutylzinnoxid zu den entsprechenden Polycarbonatdiolen umsetzen, nicht jedoch mittels schwermetallfreier Katalyse, z.B. mit basischen Salzen des Magnesiums wie Magnesiumhydroxidcarbonat. Selbst kleinste Verunreinigungen mit Salzen der Sulfonsäuren führen zu unvollständigem Aufbau des Polycarbonatdiols.

Die Verwendung organozinn-haltiger Katalysatoren ist insbesondere deswegen ein Nachteil, weil diese ökologisch nicht unbedenklich sind.

Des weiteren weisen gemäß US-A 4,808,691 hergestellte oligomere Hexandiole vergleichsweise starke Verfärbungen auf, die sich auch die entsprechenden Folgeprodukte, also Polyethercarbonatpolyole, deren Diisocyanatprepolymere sowie daraus hergestellte Polyurethanformkörper fortpflanzen. Diese unerwünschte Verfärbung stellt einen weiteren Nachteil dar.

Ein weiterer Nachteil besteht darin, dass die Hydrolyse ausschließlich mit Wasser und Ammoniak zu einer unvollständigen Hydrolyse der Sulfonsäureester führen kann. Werden diese Ester im Verlauf nachfolgender Umsetzungen gespalten, so kann die freigesetzte Sulfonsäure ebenfalls unvollständige Umsetzungen bewirken.

Aufgabe der vorliegenden Erfindung war es daher, ein Verfahren zur Herstellung von oligomeren Diolen, z.B. Hexandiolen, zur Verfügung zu stellen, welches sowohl die Herstellung deutlich vereinfacht als auch breit einsetzbare Produkte liefert, insbesondere solche, die mit basischen Magnesiumsalzen zu Polycarbonatpolyolen weiter umsetzbar sind. Zusätzlich sollten die erhaltenen oligomereren Diole und deren Folgeprodukte deutlich verminderte Verfärbungen aufweisen.

Es wurde gefunden, dass durch die Einführung eines Phasentrennungsschritts in eine organische und eine wässrige Phase bei der Aufarbeitung des Reaktionsgemischs die Abtrennung des Katalysators erleichtert wird und Produkte von verbesserter Qualität erhalten werden.

Gegenstand der Erfindung ist ein Verfahren zur Herstellung oligomerer aliphatischer Diole, bei dem
1. ein aliphatisches Diol in Gegenwart eines sauren Katalysators und eines Schleppmittels oligomerisiert und gebildetes Wasser azeoptrop abdestilliert wird,
2. die Reaktionsmischung nach Erreichen des gewünschten Oligomerisierungsgrades mit einer wässrigen Base versetzt wird und eventuell während der Oligomerisierung gebildete Ester hydrolysiert werden,
3. durch Zugabe von nicht oxidierenden anorganischer Säuren oder deren Salzen ein pH-Wert von 4,0 bis 8,0 in der Reaktionsmischung eingestellt wird, und
4. nach Phasentrennung des Reaktionsgemisches die organische Phase abgetrennt, entwässert und filtriert wird.

Als Diol werden aliphatische Diole mit einem Molekulargewicht von 100 bis 200, bevorzugt 118 bis 175 g/mol verwendet. Bevorzugt werden 1,6-Hexandiol, 3-Methyl-1,3-pentandiol, 1,7-Heptandiol, 1,8-Octandiol, 1,9-Nonandiol, oder 1,10-Decandiol eingesetzt, besonders bevorzugt 1,6-Hexandiol oder Mischungen von 1,6-Hexandiol mit bis zu 50 Gew.-% anderen Diolen aus der 3-Methyl-1,3-pentandiol, 1,7-Heptandiol, 1,8-Octandiol, 1,9-Nonandiol, und 1,10-Decandiol umfassenden Gruppe.

Als Katalysator für die Diol-Oligomerisierung werden im erfindungsgemäßen Verfahren starke Säuren mit pKs-Werten < 3 in Mengen von 0,1 bis 2 Gew.-% eingesetzt. Beispiele sind anorganische Säuren wie Schwefelsäure, Phosphorsäure, Chlorwasserstoff, Bromwasserstoff oder Iodwasserstoff bzw. deren wässrige Lösungen sowie organische Säuren wie Butansulfonsäure, Benzolsulfonsäure, Toluolsulfonsäure, Benzoldisulfonsäure und Naphthalindisulfonsäure. Bevorzugt wird Naphthalin-1,5-disulfonsäure verwendet.

Als Schleppmittel kommen Toluol, Xylol, Gasölfraktionen, Cyclohexan, oder Chlorbenzol zur Anwendung, bevorzugt ist Toluol.

Die Oligomerisierung erfolgt bevorzugt bei Temperaturen von 150 bis 200°C, einem Druck von 700 bis 1300 mbar, bevorzugt Normaldruck, über eine Dauer von 5 bis 25 Stunden, abhängig vom angestrebten Oligomerisierungsgrad, der verwendeten Menge an Katalysator sowie der Reaktionstemperatur. Bevorzugt wird die Oligomerisierungsreaktion bis zu einem Oligomerisierungsgrad von 1,5 bis 10 geführt

Im erfindungsgemäßen Verfahren erfolgt nach Ende der Oligomerisierung und azeotropen Abtrennung des Reaktionswassers eine Zugabe von wässriger anorganischer Base. Bevorzugt lässt man das Reaktionsgemisch vor Zugabe der Base auf etwa 100°C abkühlen. Als Base werden bevorzugt Alkalihydroxide, besonders bevorzugt Kalium- oder Natriumhydroxid eingesetzt. Die verwendeten Basen weisen bevorzugt einen Wassergehalt von 5 bis 50 Gew.-% auf. Die zugegebene Menge an Base beträgt bevorzugt das ein- bis zweifache der zur Neutralisation des sauren Katalysators erforderlichen stöchiometrischen Menge.

Die Hydrolyse etwaiger während der Oligomerisierung des Diols durch Reaktion des sauren Katalysators mit Hydroxylgruppen von monomeren oder oligomeren Diolen gebildeter Ester erfolgt bevorzugt durch mindestens einstündiges Rühren bei erhöhter Temperatur, unter Normaldruck oder erhöhtem Druck. Wird die Hydrolyse bei Temperaturen oberhalb 100°C durchgeführt, wird bevorzugt unter erhöhtem Druck gearbeitet. Es hat sich in der Praxis als besonders günstig erwiesen, für 2 bis 5 Std. bei 90 bis 100°C unter Normaldruck zu hydrolysieren.

Nach Beendigung der Hydrolyse wird der pH-Wert der Reaktionsmischung durch Zugabe von nicht oxidierenden anorganischer Säuren oder deren Salzen auf einen pH-Wert im Bereich von 4,0 bis 8,0, bevorzugt 4,5 bis 7,5, besonders bevorzugt 5,5 bis 7,0 eingestellt. Bevorzugt lässt man zuvor das Reaktionsgemisch auf Raumtemperatur abkühlen. Bevorzugt sind solche Säuren, deren Salze in Wasser gut löslich sind. Bevorzugt sind Schwefelsäure und deren saure Alkalisalze, sowie analoge Phosphorsäure- und Kohlensäureverbindungen. Die Säuren bzw. deren Salze können in reiner Form oder als Lösung in einem Lösungsmittel, insbesondere Wasser, zur Anwendung kommen.

Der pH-Wert wird vorzugsweise bestimmt, indem man 20 ml eines Gemisches aus 9 Teilen Methanol und 1 Teil Wasser mittels 1/100N NaOH oder KOH auf pH 7,0 einstellt, dann 10 ml der Reaktionsmischung einrührt und nach 5 Minuten den pH-Wert abliest. Hierfür kann ein handelsübliches pH-Meter mit pH-Elektrode verwendet werden.

Ist ein pH-Wert von 5,5 bis 7,0 erreicht, stellt man das Rührwerk ab und wartet die Phasentrennung in obere organische und untere wässrige Phase ab. In einer bevorzugten Variante können zur Beschleunigung der Phasentrennung nach erfolgter pH-Einstellung in Wasser leicht lösliche Salze, bevorzugt Kochsalz, zugesetzt werden.

Die wässrige Phase wird bei 30 - 50°C abgetrennt und die verbleibende organische Phase unter vermindertem Druck (0,001 - 100 mbar) bei erhöhter Temperatur (90 - 160°C) von Restmengen Wasser und in der Kondensationsphase verwendetem Schleppmittel befreit.

Nach Abkühlen auf 60 - 100°C, bevorzugt 75 - 85°C wird die organische Phase über eine Filterdrucknutsche, belegt mit einem feinporigen Filterpapier (z.B. Suprasec-1000 der Fa. Seitz), filtriert. Man erhält eine wasserklare, leicht gelbliche Flüssigkeit. Die Filtrationszeit für einen 3 Tonnen-Ansatz beträgt ca. 3-4 Stunden.

Die erfindungsgemäß erhaltenen oligomeren Diole eignen sich besonders zur Herstellung von Polycarbonatdiolen. Gegenstand der Erfindung ist daher auch ein Verfahren zur Herstellung von Polycarbonatdiolen, bei dem ein nach dem erfindungsgemäßen Verfahren erhaltenes oligomeres Diol, gegebenenfalls nach Zusatz von monomerem Diol, insbesondere 1,6-Hexandiol zur Einstellung des Oligomerisierungsgrades, mit einer unterstöchiometrischen Menge eines Carbonatspenders wie Diphenylcarbonat, Dimethylcarbonat oder Phosgen in Gegenwart eines Katalysators umgesetzt wird. Bevorzugt findet als Carbonatspender Diphenylcarbonat Verwendung.

Das Molekulargewicht des Polycarbonatdiols kann hierbei durch Variation der stöchiometrischen Einsatzverhältnisse von oligomerem Diol, monomerem Diol und Carbonatspender in weiten Grenzen variiert werden. Bevorzugt sind jedoch Polycarbonatdiole mit zahlenmittleren Molekulargewichten von 800 bis 2800, besonders bevorzugt von 1500 bis 2500 g/mol.

Als Katalysator werden bevorzugt schwermetallfreie Katalysatoren eingesetzt. Besonders bevorzugt sind Magnesiumsalze, insbesondere Magnesiumhydroxidcarbonat. Weitere Einzelheiten des Herstellverfahrens der erfindungsgemäßen Polycarbonatpolyole sind in DE-OS 101 25 557 offenbart.

Wird die Reaktion in Gegenwart eines basischen Magnesiumsalzes als Katalysator durchgeführt, wird dieser anschließend durch Zugabe von Säure neutralisiert. Beispiele für zu verwendende Säuren sind Weinsäure, Zitronensäure, Dibutylphosphat, Phosphorsäure, Schwefelsäure und deren saure Salze. Bevorzugt wird zur Neutralisation des basischen Magnesium-Katalysators Schwefelsäure eingesetzt, wobei eine klare Lösung erhalten wird. Eine Abtrennung des gebildeten Magnesiumsulfats aus dem Produkt ist nicht erforderlich, da dieses sich bei der weiteren Umsetzung des Polycarbonatdiols inert verhält.

Ein weiterer Gegenstand der Erfindung ist ein Verfahren zur Herstellung NCO-terminierter Prepolymere aus den erfindungsgemäß erhaltenen Polycarbonatdiolen. Hierbei wird ein nach dem erfindungsgemäßen Verfahren erhaltenes Polycarbonatdiol in unterstöchiometrischer Menge mit einem Polyisocyanat umgesetzt. Als Polyisocyanat werden bevorzugt Diisocyanate aus der Diphenylmethandiisocyanat, Naphthalin-1,4-diisocyanat, Naphthalin-1,5-diisocyanat, Duroldiisocyanat, Toluylendiisocyanat, Hexamethylen-1,6-diisocyanat und Isophorondiisocyanat umfassenden Gruppe eingesetzt. Besonders bevorzugt sind 4,4'-Diphenylmethandiisocyanat (z.B. Desmodur® 44M, Bayer AG) sowie dessen Gemische mit 2,4'- und 2,2'-Diphenylmethandiisocyanat, wobei deren Anteile unter 10 Gew.-% betragen. Die erfindungsgemäßen Prepolymere zeichnen sich gegenüber denen des Standes der Technik durch eine wesentlich verbesserte Lagerstabilität aus.

### Beispiele

### Herstellung oligomerer Hexandiole

### Beispiel 1

240 kg (2069 Mol) aufgeschmolzenes Hexandiol wurden in einem 300-Liter VA-Kessel mit Kolonne und Azeotrop-Aufsatz, Rückflusskühler und Destillatvorlage unter Rühren mit 2,5 kg wässriger 1,5-Naphthalindisulfonsäure (35 Gew.-%ig) und 10 kg Toluol vermischt. Man erhöhte die Temperatur auf 170°C und destillierte im Verlauf von 10 Std. unter leichtem Stickstoffstrom 22 kg Wasser ab.

Man kühlte auf 100°C ab, evakuierte und zog mit Restvakuum 10 kg destilliertes Wasser ein. Nach Abkühlen des Kesselinhaltes auf 30°C wurden 0,908 kg wässrige Natronlauge (32 Gew.-%ig) zugegeben und im Verlauf von 2 Std. auf 100°C erhitzt. Man rührte 1 Std. bei 100°C nach, kühlte auf 50°C ab und gab 103 g konzentrierte wässrige Natriumhydrogensulfatlösung zu und rührte für weitere 30 min. bei dieser Temperatur. Der pH-Wert der Reaktionsmischung betrug 6,4, die Säurezahl 0,05 mg KOH/g.

Anschließend wurde auf 30°C abgekühlt und es wurden 44 kg wässrige Kochsalzlösung (10 Gew.-%ig) intensiv eingerührt. Nach Abstellen des Rührers trennten sich die Phasen in ca. 30 min; die untere Phase wurde abgelassen. Das im Kessel verbliebene Produkt wurde durch Anlegen von Vakuum (1 mbar) bei 140°C für 3 Std. entwässert und auf 80°C abgekühlt. Man filtrierte in 40 Minuten über ein mit einer Supra 5500 Filterplatte Seitzfilter.

Die Ausbeute betrug 190 kg, der Wassergehalt des Produktes 0,02 Gew.-%, die Hydroxylzahl 466 mg KOH/g, die Säurezahl 0,05 mg KOH/g.

### Beispiel 2 (Vergleich)

240 kg (2069 Mol) aufgeschmolzene Hexandiol wurden in einem 300-Liter VA-Kessel mit Kolonne und Azeotrop-Aufsatz, Rückflusskühler und Destillatvorlage unter Rühren mit 2,5 kg wässriger 1,5-Naphthalindisulfonsäure (35 Gew.-%ig) und 10 kg Toluol vermischt. Man erhöhte die Temperatur auf 170°C und destillierte im Verlauf von 10 Std. unter leichtem Stickstoffstrom 21,105 kg Wasser ab. Die experimentell bestimmte Hydroxylzahl betrug zu diesem Zeitpunkt 431 mg KOH/g.

Man kühlte auf 100°C ab, evakuierte und zog mit Restvakuum 10 kg destilliertes Wasser ein. Nach Abkühlen des Kesselinhaltes auf 30°C wurden 0,9 kg Ammoniakwasser zugegeben und es wurde im Verlauf von 3 Std. auf 80°C erhitzt. Man legte nach Erreichen dieser Temperatur langsam Vakuum an, wobei bei 350 mbar die Destillation von Wasser einsetzte. Bei Erreichen von 15 mbar wurde die Temperatur schrittweise auf 140°C erhöht und der Druck mittels Ölpumpe auf 2 mbar abgesenkt. Unter diesen Bedingungen rührte man eine weitere Stunde nach, verminderte anschließend die Temperatur auf 80°C und belüftete den Kessel. Man filtrierte in 320 Minuten über ein mit einer Supra 5500 Filterplatte Seitzfilter, wobei nach jeweils ca. 45 Minuten ein Filterwechsel erforderlich war.

Die Ausbeute betrug 201 kg, der Wassergehalt des Produktes 0,02 Gew.-%, die Hydroxylzahl 486 mg KOH/g, die Säurezahl 0,05 mg KOH/g.

### Herstellung von Polycarbonatdiolen

### Beispiel 3

3000 g des in Beispiel 1 hergestellten oligomeren Hexandiols wurden mit 1107 g Hexandiol und 4157 g Diphenylcarbonat in Gegenwart von 150 mg Magnesiumcarbonathydroxid-pentahydrat umgesetzt. Hierbei erhitzte man das Reaktionsgemisch für eine Stunde auf 180°C, kühlte auf 120°C ab und steigerte die Temperatur im Verlauf von 6 Stunden auf 200°C, wobei der Druck ab ca. 120°C 15 mbar betrug. Zur Vervollständigung der Reaktion wurde bei 200°C und einem Druck von < 1 mbar für 2 Std. nachgerührt. Man destillierte insgesamt 3652 g Phenol ab. Zur Neutralisation des basischen Magnesiumkatalysators wurden 175mg konzentrierte Schwefelsäure zugesetzt. Man erhielt 4600 g Polycarbonatdiol.

Die OH-Zahl des Polycarbonatdiols betrug 57,9 mg KOH/g, die Säurezahl 0,12 mg KOH/g, die Viskosität (nach DIN 53015) 1100 mPas (75°C). Die UV-spektroskopische Bestimmung der Endgruppen ergab einen Gehalt von < 0,01Gew.-% Phenylcarbonatogruppen, 0,14 Gew.-% Phenoxygruppen, sowie 0,02 Gew.-% freies Phenol.

### Beispiel 4 (Vergleich)

1800 g oligomeres Hexandiol mit einer OH-Zahl von 501 mg KOH/g, hergestellt nach dem in US-A 4,808,691 beschriebenen Verfahren, wurden mit 448 g Hexandiol und 2274 g Diphenylcarbonat in Gegenwart von 70 mg Dibutylzinnoxid umgesetzt. Hierbei erhitzte man das Reaktionsgemisch für eine Stunde auf 180°C, kühlte auf 120°C ab und steigerte die Temperatur im Verlauf von 6 Stunden auf 200°C, wobei der Druck ab ca. 120°C 15 mbar betrug. Zur Vervollständigung der Reaktion wurde bei 200°C und einem Druck von < 1 mbar für 2 Std. nachgerührt. Man destillierte insgesamt 2002 g (mit Hexandiol verunreinigtes) Phenol ab und erhielt 2520 g Polycarbonatpolyol mit einer OH-Zahl von 48,0 mg KOH/g.

Durch Zusatz von 22,5 g Hexandiol und dessen Einesterung wurde die OH-Zahl des Polyethercarbonatpolyols auf 56,6 mg KOH/g erhöht. Die Säurezahl des Produkts betrug 0,2 mg KOH/g, die Viskosität (nach DIN 53015) 900 mPas (75°C), die Farbzahl >999 Hazen. Der Hexandiolnachsatz ergab somit die angestrebte OH-Zahl, dennoch war die Phenolabspaltung unvollständig. Folge hiervon ist die deutlich und unerwünschterweise nach unten abweichende Viskosität.

### Beispiel 5 (Vergleich)

1556 g oligomeres Hexandiol mit einer OH-Zahl von 501 mg KOH/g, hergestellt nach dem in US-A 4,808,691 beschriebenen Verfahren, wurden mit 426 g Hexandiol und 2005 g Diphenylcarbonat in Gegenwart von 358mg Magnesiumcarbonathydroxid-pentahydrat umgesetzt. Hierbei erhitzte man das Reaktionsgemisch für eine Stunde auf 180°C, kühlte auf 120°C ab und steigerte die Temperatur im Verlauf von 6 Stunden auf 200°C, wobei der Druck ab ca. 120°C 15 mbar betrug. Es konnte kein Phenol abdestilliert werden.

Herstellung von NCO-terminierten Prepolymeren

### Beispiel 6 (Vergleich)

781,8 kg reines Diphenylmethandiisocyanat (Desmodur® 44 M, Bayer AG) wurden mit 1300 kg eines gemäß (Vergleichs-)Beispiel 4 hergestellten Polycarbonatdiols zwei Stunden lang bei 80°C zur Reaktion gebracht. Man erhielt ein NCO-terminiertes Prepolymer mit einem NCO-Gehalt (nach DIN 53185) von 9,95 Gew.-% und einer Viskosität von 1920 mPas (70°C) (nach DIN 53015).

Zur Bestimmung der Lagerstabilität wurde eine Probe des Prepolymers drei Tage lang unter N₂ bei 80°C im Trockenschrank gelagert. Nach dieser Lagerung war die Viskosität um ca. 26 % auf 2420 mPas (70°C) angestiegen und der NCO-Gehalt auf 9,73 Gew.-% gesunken.

### Beispiel 7

41,4 kg reines Diphenylmethandiisocyanat (Desmodur® 44 M, Bayer AG) wurden mit 68,6 kg eines gemäß Beispiel 3 hergestellten Polycarbonatdiols zwei Stunden lang bei 80°C zur Reaktion gebracht. Man erhielt ein NCO-terminiertes Prepolymer mit einem NCO-Gehalt (nach DIN 53185) von 9,91 Gew.-% und einer Viskosität von 1910 mPas (70°C) (nach DIN 53015).

Zur Bestimmung der Lagerstabilität wurde eine Probe des Prepolymers drei Tage lang unter N₂ bei 80°C im Trockenschrank gelagert. Nach dieser Lagerung war die Viskosität um ca. 9,5 % auf 2090 mPas (70°C) angestiegen und der NCO-Gehalt auf 9,77 Gew.-% gesunken.

Die Lagerung drei Tage bei 80°C simuliert eine Lagerung bei RT über einen längeren Zeitraum (ca. 6 Monate, s.a. G.M. Barrow: _{"}Physikalische Chemie", Teil III, Vieweg Verlag, Braunschweig 1970, S.296). Die durchgeführten Messungen zeigen, dass Prepolymere, die aus den erfindungsgemäßen Polycarbonatdiolen hergestellt wurden, um den Faktor 3 lagerstabiler sind als konventionell hergestellte Prepolymere.

## Patentansprüche

1. Verfahren zur Herstellung oligomerer aliphatischer Diole, bei dem die Aufarbeitung des Reaktionsgemischs einen Phasentrennungsschritt in eine organische und eine wässrige Phase umfasst.

2. Verfahren zur Herstellung oligomerer aliphatischer Diole, bei dem
1. ein aliphatisches Diol in Gegenwart eines sauren Katalysators und eines Schleppmittels oligomerisiert und gebildetes Wasser azeoptrop abdestilliert wird,
2. die Reaktionsmischung nach Erreichen des gewünschten Oligomerisierungsgrades mit einer wässrigen Base versetzt wird und eventuell während der Oligomerisierung gebildete Ester hydrolysiert werden,
3. durch Zugabe von nicht oxidierenden anorganischer Säuren oder deren Salzen ein pH-Wert von 4,0 bis 8,0 in der Reaktionsmischung eingestellt wird, und
4. nach Phasentrennung des Reaktionsgemisches die organische Phase abgetrennt, entwässert und filtriert wird.

3. Verfahren zur Herstellung von Polycarbonatdiolen, bei dem ein gemäß Anspruch 1 oder 2 hergestelltes oligomeres aliphatisches Diol mit einer unterstöchiometrischen Menge eines Carbonatspenders in Gegenwart eines Katalysators umgesetzt wird.

4. Verfahren gemäß Anspruch 3, bei dem als Katalysator ein basisches Magnesiumsalz eingesetzt wird.

5. Verfahren gemäß Anspruch 3 oder 4, bei dem als Carbonatspender Diphenylcarbonat eingesetzt wird.

6. Verfahren zur Herstellung NCO-terminierter Prepolymere, bei dem ein gemäß Anspruch 4 oder 5 erhaltenes Polycarbonatdiol in unterstöchiometrischer Menge mit einem Polyisocyanat umgesetzt wird.

7. Verfahren gemäß Anspruch 6, bei dem als Polyisocyanat Diphenylmethandiisocyanat eingesetzt wird.

8. Verwendung der gemäß Anspruch 1 oder 2 erhältlichen oligomeren aliphatischen Diole zur Herstellung von Polyurethanen.

9. Verwendung der gemäß einem der Ansprüche 3 bis 5 erhältlichen Polycarbonatdiole zur Herstellung von Polyurethanen.

10. Verwendung der gemäß einem der Ansprüche 6 bis 8 erhältlichen NCO-terminierten Prepolymere zur Herstellung von Polyurethanen.
